# EUROPEAN PATENT APPLICATION

(11) **EP 4 520 752 A1**
(43) Date of publication of application: **12.03.2025**
(21) Application number: 23799169.0
(22) Date of filing: 14.04.2023
(51) Int. Cl.: C07D 211/46, A61P 37/08, A61P 11/02, A61P 17/00, A61K 31/445

(54) **CAREBASTINE SALT AND USE THEREOF**

(30) Priority: 06.05.2022 CN 202210486129
(71) Applicant: Chengdu Shibeikang Biomedical Technology Co., Ltd., Chengdu, Sichuan 611731 (CN)
(72) Inventor: ZENG, Yanqun, Chengdu, Sichuan 611731 (CN); ZHOU, Guanglin, Chengdu, Sichuan 611731 (CN); FU, Haixia, Chengdu, Sichuan 611731 (CN); MOU, Xia, Chengdu, Sichuan 611731 (CN)
(74) Representative: Herrero & Asociados, S.L.
(86) International application number: PCT/CN2023/088517
(87) International publication number: WO 2023/213182

(57) **Abstract**

The present invention discloses a carebastine salt and a use of the carebastine salt, relates to the field of pharmaceutical chemistry, and solves the problems of carebastine in the related art such as poor solid form, excessive impurities, instability, difficulty in purification, difficulty in scale-up synthesis, and unsuitability for medicinal use. The carebastine salt of the present invention includes, but is not limited to, acid addition salts or base addition salts, and particularly includes potassium salts, sodium salts, methanesulfonate and p-toluenesulfonate. The carebastine salt of the present invention has the use of preparing histamine H1 receptor antagonist drugs. The carebastine salt of the present invention has the characteristics such as easy purification, high stability, simple process and easy industrial production, and has good hygroscopicity characteristics and is convenient for storage. At the same time, the salt of the present invention can quickly enter the body to exert the efficacy, has good oral absorption, is superior to ebastine in safety and individual differences, and is a promising anti-allergic disease drug.

## Description

This application claims the priority to an invention patent filed on May 6, 2022 with the title of "Carebastine salt and use of same" and the patent number CN202210486129.2, which is hereby expressly incorporated herein by reference.

### TECHNICAL FIELD

The present invention relates to the technical field of pharmaceutical chemistry, and in particular to a carebastine salt and a use of the carebastine salt.

### BACKGROUND OF THE INVENTION

Allergic disease, also known as hypersensitiveness disease, refers to a disease caused by allergic reactions after contact with allergens. At present, common allergic diseases in clinical practice mainly include allergic rhinitis, asthma, urticaria, atopic dermatitis, allergic conjunctivitis, and allergic gastrointestinal diseases, and the like. Allergic reaction refers to the reaction of tissue damage or dysfunction when an immune body is stimulated by the same antigen again. In recent years, the incidence of allergic disease in China has continued to rise, which is close to 40%. Take allergic rhinitis as an example, allergic rhinitis is the disease with the highest incidence. According to incomplete statistics, the number of patients worldwide has exceeded 500 million. Allergic rhinitis is mainly manifested by nasal congestion, nasal itching, runny nose, sneezing, and the like. The symptoms of an allergic disease recur over a long period of time. These symptoms may affect the patient's quality of life, including sleep, social interaction, and work if timely prevention or intervention is not performed.

Histamine H1 receptor antagonists are a class of drugs commonly used to prevent and treat an allergic disease. From the perspective of development, the histamine H1 receptor antagonists are mainly divided into three generations. The first-generation drugs, such as chlorpheniramine, promethazine, diphenhydramine, and cyproheptadine, have short-acting effects, and central depressant and sedative effects, and show adverse reactions such as drowsiness, epilepsy, rash, and anaphylactic shock. The second-generation drugs, such as loratadine, cetirizine, ebastine, astemizole and terfenadine, have long-acting and non-sedative effects. Among these histamine H1 receptor antagonists, astemizole and terfenadine have been found to prolong the QTc interval and, in rare cases, cause arrhythmias in overdose or other specific conditions. The regulatory authorities have now revoked the approval of astemizole and terfenadine. The third-generation drugs are represented by mizolastine, desloratadine and levocetirizine. Among these histamine H1 receptor antagonists, mizolastine has a unique dual effect of antihistamine and anti-other inflammatory mediators. Desloratadine is an active metabolite of loratadine, which solves the problem of large individual differences in loratadine. Levocetirizine is a single isomer of cetirizine.

Ebastine tablets belong to the second-generation histamine H1 receptor antagonists, which are potent, long-acting, highly selective histamine H1 receptor blockers and have no antagonistic effect on cholinergic receptors of the central nervous system. Ebastine tablets can selectively block histamine H1 receptors without sedation effect, and are used to treat various types of allergic diseases such as urticaria, allergic rhinitis, eczema, dermatitis, pruritus and pruritus. After oral administration, ebastine is rapidly absorbed and most of ebastine is metabolized by the strong first-pass effect in the liver. Ebastine is metabolized mainly through two routes. One is dealkylation under the action of CYP3A enzymes to generate dealkylated ebastine metabolites. The other is to generate hydroxy ebastine metabolites under the action of CYP2J2 enzymes, and hydroxy ebastine is then oxidized by CYP2J2 and CYP3A4 enzymes to generate an acidic metabolite, carebastine. Ebastine and a metabolite thereof, carebastine, do not enter the central nervous system and have no adverse central nervous system and anticholinergic effects.

There has been no report and no evidence of QTc interval prolongation associated with treatment with ebastine. Therefore, once a day of ebastine administration provides an effective and well-tolerated alternative to other second-generation antihistamines currently used as first-line treatment for seasonal and perennial allergic rhinitis and chronic idiopathic urticaria. Compared with the first-generation H1 receptor antagonists, ebastine has no obvious cardiac and psychomotor system abnormalities. However, since the oral onset time of ebastine is 1 to 4 hours, ebastine is not suitable for the emergency treatment of an acute allergic disease such as acute urticaria and asthma.

Currently, the only fast-acting histamine H1 receptor antagonists in clinical practice are levocetirizine and cetirizine. Compared with ebastine, levocetirizine and cetirizine are easier to enter the central nervous system, and clinical manifestations include drowsiness and headache. Therefore, there is an urgent clinical need for non-sedative, fast-acting histamine H1 receptor antagonists that can be used in patients with an acute allergic disease such as acute urticaria and asthma.

Carebastine is the in vivo active metabolite of ebastine, and its structural formula is shown in Formula I:

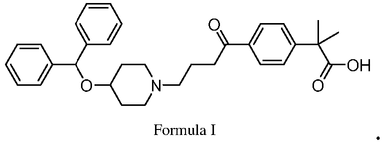

Like ebastine, carebastine has a strong selective antagonistic effect on histamine H1 receptors, can inhibit the release of histamine, and has weak antagonistic effect on H1 receptors and anticholinergic effect of the central nervous system. Direct use of carebastine as medicine can effectively solve the problem of slow oral onset time of ebastine. However, carebastine does not have a good solid form, which makes it unable to meet pharmaceutical requirements. Preparations with poor solid form cannot be used stably, are difficult to purify, cannot be scaled up for production, and have uncontrollable quality, making them unsuitable for medicinal use. Therefore, there is currently no carebastine drug on the market.

### SUMMARY OF THE INVENTION

An object of the present invention is to provide a carebastine salt to solve the problems of carebastine in the related art, such as poor solid form, excessive impurities, instability, difficulty in purification, difficulty in scale-up synthesis, and unsuitability for medicinal use.

The second object of the present invention is to provide a use of the carebastine salt.

The third object of the present invention is to provide a composition comprising the carebastine salt.

In order to achieve the above objects, the present invention provides the following technical solutions.

In one aspect, the present invention provides a pharmaceutically acceptable salt of carebastine, which is an acid addition salt formed by carebastine and an acid or a base addition salt formed by carebastine and a base.

In some embodiments of the present invention, the acid addition salt is an inorganic acid addition salt, or an organic acid addition salt.

Furthermore, the inorganic acid addition salt is selected from sulfate, bisulfate, nitrate, hydrobromides, hydroiodide, carbonate, bicarbonate, sulfite, bisulfite, pyrosulfate, monohydrogen phosphate, dihydrogen phosphate, perchlorate, persulfate, hemisulfate, bisulfate, thiocyanate, phosphate, pyrophosphate or metaphosphate.

Furthermore, the organic acid addition salt is selected from formate, acetate, propionate, butyrate, benzoate, malonate, succinate, pyruvate, methanesulfonate, ethanesulfonate, propanesulfonate, citrate, 4-nitrobenzoate, benzenesulfonate, p-toluenesulfonate, malate, propiolate, 2-butynoate, 2-hydroxy-ethanesulfonate, vinylacetate, tartrate, L-tartrate, fumarate, isethionate, maleate, lactate, lactobionate, pamoate, salicylate, galactarate, glucoheptonate, mandelate, 1,2-ethanedisulfonate, oxalate, trifluoroacetate, trifluoromethanesulfonate, adipate, suberate, sebacate, butyne-1,4-dicarboxylate, hexyne-1,6- dicarboxylate, glycolate, alginate, ascorbate, isoascorbate, aspartate, L-aspartate, glutamate, L-glutamate, 2-phenoxybenzoate, 2-(4-hydroxybenzoyl)benzoate, acetoacetate, 2-hydroxyethanesulfonate, borate, chlorobenzoate, camphorate, itaconate, camphorsulfonate, L-camphorsulfonate, methylbenzoate, dinitrobenzoate, sulfamate, lactobionate, galacturonate, cyclopentylpropionate, dodecyl sulfate, acrylate, cyclopentanepropionate, glycerophosphate, methoxybenzoate, digluconate, gluconate, heptanoate, hexanoate, 2-hydroxy-ethanesulfonate, trimethylacetate, glucuronate, laurate, phthalate, phenylacetate, lauryl sulfate, 2-acetoxybenzoate, nicotinate, cinnamate, oleate, palmitate, pectinate, phthalate, glutarate, hydroxymaleate, hydroxybenzoate, phenylacetate, 3-hydroxy-2-naphthoate, 3-phenylpropionate, isobutyrate, pivalate, picrate, stearate, 2,2-dichloroacetate, acylated amino acid salts, alginate, 4-acetamidobenzenesulfonate, caprate, cholate, octanoate, nonanoate, cyclamate, phthalate, cysteine hydrochloride, sorbate, pamoate, mucate, glycinate hydrochloride, naphthalene disulfonate, xylene sulfonate, cystine dihydrochloride, undecanoate, polyvinyl sulfonate, sulfosalicylate, phenylbutyrate, 4-hydroxybutyrate, polyvinyl sulfate, naphthalene-1-sulfonate, naphthalene-2-sulfonate or valerate.

In some embodiments of the present invention, the organic acid addition salt is selected from methanesulfonate, benzenesulfonate, p-toluenesulfonate, naphthalene disulfonate, naphthalene-1-sulfonate, or naphthalene-2-sulfonate.

Furthermore, the organic acid addition salt is selected from methanesulfonate, benzenesulfonate or p-toluenesulfonate.

In some embodiments of the present invention, the base addition salt is selected from an alkali metal salt, a substituted or unsubstituted ammonium salt, an amine salt, an alkaline amino acid salt, or a substituted or unsubstituted pyridine salt.

Furthermore, the metal base addition salt is selected from a lithium salt, a sodium salt, a potassium salt, a calcium salt, a magnesium salt, an aluminum salt, an iron salt or a zinc salt, preferably a potassium salt or a sodium salt.

Furthermore, the substituted or unsubstituted ammonium salt is selected from an ammonium salt, a tetramethylammonium salt, a tetraethylammonium salt or a choline salt.

Furthermore, the amine salt is selected from methylamine salts, dimethylamine salts, trimethylamine salts, ethylamine salts, diethylamine salts, triethylamine salts, isopropylamine salts, tert-butylamine salts, diisopropylamine salts, 2-ethylaminoethanol salts, tromethamine salts, ethanolamine salts, diethanolamine salts, triethanolamine salts, piperidine salts, piperazine salts, morpholine salts, meglumine salts, N-methylglucosamine salts, dimethylglucosamine salts, ethylglucosamine salts, dicyclohexylamine salts, 1,6-hexanediamine salts, galactosamine salts, glucosamine salts, trishydroxymethylaminomethane salts, aminopropanediol salts, 1-amino-2,3,4-butanetriol salts or serinol salts.

Furthermore, the alkaline amino acid salt is selected from lysine salts, arginine salts, L-arginine salts, histidine salts, L-histidine salts, sarcosine salts, L-lysine salts or ornithine salts.

Furthermore, the substituted or unsubstituted pyridine salt is selected from pyridinium salts, methyl pyridinium salts, ethyl pyridinium salts or propyl pyridinium salts.

In some embodiments of the present invention, the carebastine salt is a single salt or a double salt, in a single salt, the molar ratio of carebastine to counter-ion is 1:1 to 2:1, and in a double salt, the molar ratio of carebastine to counter-ion is 1:4 to 4:1.

Furthermore, in some embodiments, the carebastine salt is selected from

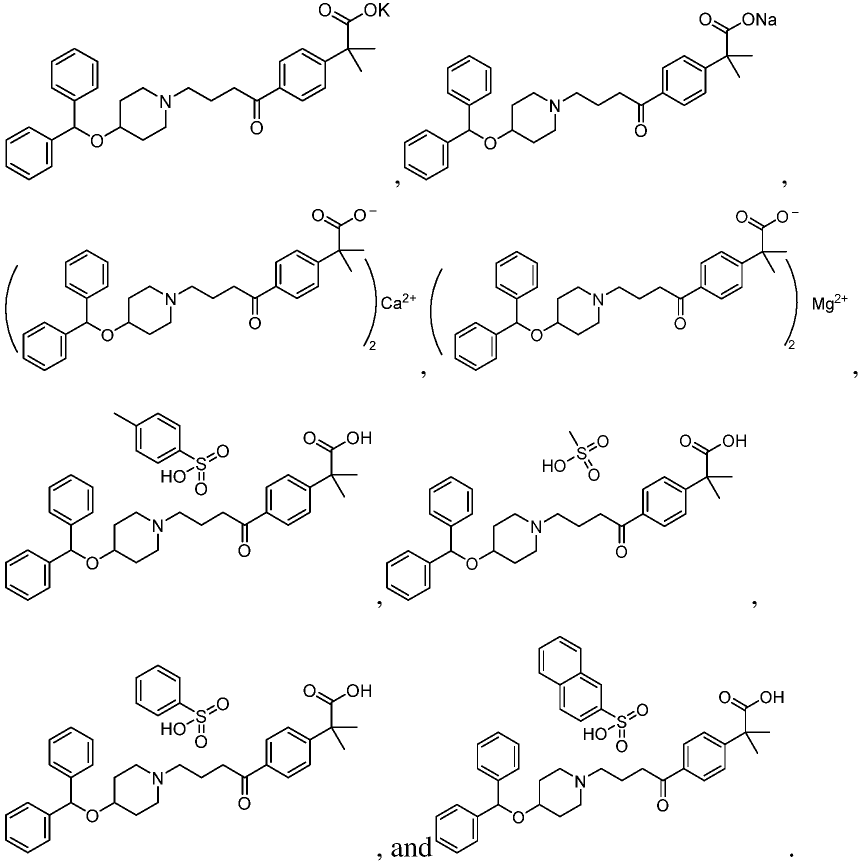

In another aspect, the present invention provides a method for preparing the carebastine salt according to any one of the above items, the method including: heating 2-(4-(4-(4- (diphenylmethoxy) piperidin-1-yl) butyryl) phenyl) -2-methylpropanoic acid with an acid or a base in an organic solvent to form a salt.

Furthermore, the above acid includes an organic acid or an inorganic acid. Preferably, the organic acid includes, but is not limited to, p-toluenesulfonic acid, methanesulfonic acid, benzenesulfonic acid or naphthalene-2-sulfonic acid.

Furthermore, the above base includes a metal-based alkali. Preferably, the metal-based alkali includes, but is not limited to, sodium hydroxide, potassium hydroxide, calcium hydroxide or magnesium hydroxide.

Furthermore, the organic solvent includes, but is not limited to, alcohols, esters or nitriles. Preferably, the alcohols include, but are not limited to, methanol, ethanol or isopropanol, the esters include, but are not limited to, ethyl acetate, and the nitriles include, but are not limited to, acetonitrile.

Furthermore, the heating temperature is selected from 30°C to 100°C, preferably 50°C to 60°C.

In a third aspect, the present invention provides a pharmaceutical composition of the carebastine salt according to any one of the above items, in which the pharmaceutical composition further includes a pharmaceutically acceptable carrier.

The "pharmaceutically acceptable carrier" refers to a diluent, adjuvant, excipient or vehicle that is administered together with active ingredients, and is suitable, within the scope of reasonable medical judgment, for contact with tissues of humans and/or other animals without excessive toxicity, irritation, allergic response, or other problems or complications commensurate with a reasonable benefit/risk ratio.

In a fourth aspect, the present invention provides a use of the carebastine salt according to any one of the above items in preparation of a histamine H1 receptor antagonist.

Furthermore, the use includes a use of the carebastine salt according to any one of the above items in preparation of a drug for treating and/or preventing an allergic disease.

Furthermore, the allergic disease is an acute allergic disease.

Furthermore, the allergic disease is selected from urticaria, allergic rhinitis, eczema, dermatitis or pruritus.

Furthermore, the acute allergic disease is acute urticaria or acute allergic rhinitis.

In the present invention, the English names represented by the abbreviations are as follows.
DMAC: acetamide
EA: ethyl acetate
DMSO: dimethyl sulfoxide
CMC-Na: sodium carboxymethyl cellulose

### Advantageous Effects

Compared with the related art, the present invention has the following beneficial effects.

The carebastine salt of the present invention is a white powdery solid having a good solid form for pharmaceutical use, high purity, and high stability. Carebastine is an active metabolite of ebastine, can produce pharmacological effects without enzymatic metabolism after entering the body, takes effect rapidly, and avoids the safety risks caused by individual medication differences due to genetic polymorphisms in enzymes, as well as the risk of continuous life-threatening allergies caused by ineffective treatment. The carebastine salt of the present invention provides a new clinical option for allergic patients.

Carebastine is an acid-base amphoteric compound. The applicant has systematically studied the carebastine salts after a large number of experiments and found that the salts that carebastine can form are limited, and the purity and stability of acid addition salts are better than those of base addition salts, such as the purity and stability of p-toluenesulfonate are better than those of the potassium salt. However, the inventors have found that the acid addition salts of carebastine have a more serious problem in terms of hygroscopicity. In comparison, the potassium salt and p-toluenesulfonate of the present invention have better hygroscopicity characteristics.

On the other hand, the solubility of p-toluenesulfonate in the present invention is not as good as that of free carebastine. However, from the pharmacokinetic study of rats, the applicants surprisingly found that under the same molar administration, both p-toluenesulfonate and potassium salt of carebastine have better pharmacokinetic properties than ebastine and free carebastine. For example, in comparison of AUC, the AUC of carebastine in vivo in the p-toluenesulfonate group and the potassium salt group of carebastine is significantly greater than that in the ebastine group and the free carebastine group, and Tₘₐₓ of the generated carebastine is less than Tₘₐₓ of ebastine, indicating that the carebastine salt of the present invention have a faster onset of action and has better pharmacokinetic properties under the same molar administration. In addition, through acute toxicity studies on mice, the applicants found that the acute toxicity of the p-toluenesulfonate and potassium salt of carebastine is lower than that of ebastine.

### BRIEF DESCRIPTION OF THE DRAWINGS

FIG. 1 is a HPLC chromatogram of carebastine (free state) on day 0.
FIG. 2 is a HPLC chromatogram of carebastine (free state) under light (day 12).
FIG. 3 is a HPLC chromatogram of carebastine p-toluenesulfonate on day 0.
FIG. 4 is a HPLC chromatogram of carebastine p-toluenesulfonate under light (day 12).

### DETAILED DESCRIPTION OF THE EMBODIMENTS

The present invention will be further described in detail below with reference to examples and experimental examples. The embodiments and experimental examples of the present invention are only used to illustrate the technical scheme of the present invention, and are not intended to limit the present invention. Any equivalent substitutions in the art made in accordance with the contents disclosed in the present invention shall fall within the protection scope of the present invention.

The structures of the compounds were determined by nuclear magnetic resonance (¹H NMR) or liquid chromatography-mass spectrometry (LC-MS).

The LC/MS instrument (LC-MS) is Agilent G6120B (used with Agilent 1260 liquid phase), the nuclear magnetic resonance instrument (¹H NMR) is Bruker AVANCE-400 or Bruker AVANCE-800, the nuclear magnetic resonance (¹H NMR) shift (δ) is given in parts per million (ppm), the internal standard is tetramethylsilane (TMS), and the chemical shift is given in 10⁻⁶ (ppm).

The term "room temperature" in the present invention means a temperature between 10°C and 30°C.

### Example 1: Preparation of potassium 2-(4-(4-(4-(diphenylmethoxy)piperidin-1-yl)butyryl)phenyl)-2-methylpropanoate (carebastine potassium salt)

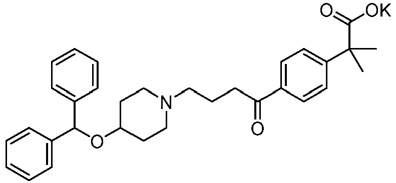

### Step 1: Preparation of methyl 2-(4-(4-(4-(diphenylmethoxy) piperidin-1-yl) butanoyl) phenyl) -2-methylpropanoate

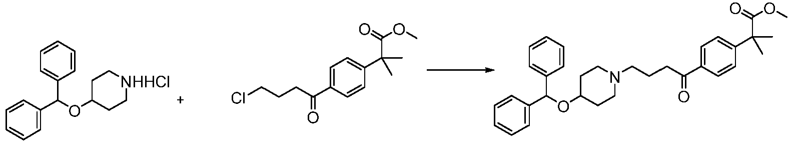

In a 25 ml single-necked flask, 4-(diphenylmethoxy)piperidine hydrochloride (473 mg, 1.77 mmol), DMAC (4.5 ml), K₃PO₄ (1.13 g, 5.3 mmol), and KI (29 mg, 0.177 mmol) were added and heated to 100°C under stirring. In 1ml of DMAC, methyl 2-(4-(4-chlorobutanoyl)phenyl)-2-methylpropanoate (600 mg, 2.12 mmol) was dissolved, followed by slowly adding the reaction solution dropwise. The reaction was carried out for 4 h to 6 h under heat preservation. The completion of the reaction of the raw materials was detected by TLC. The temperature was lowered to the room temperature, isopropyl acetate and water were added, and the mixture was stirred to separate the layers. The aqueous phase was extracted with isopropyl acetate, and the organic phases were combined, washed twice with water, dried over anhydrous sodium sulfate, filtered, concentrated, and passed through a silica gel column to obtain 500 mg of methyl 2-(4-(4-(4-(diphenylmethoxy) piperidin-1-yl) butanoyl) phenyl) -2-methylpropanoate with a yield of 45% and a purity of 97.3%.

ESI-MS: *m*/*z* = 514.3(M+H)⁺.

¹H NMR (400 MHz, CDCl₃) δ: 7.93 (d, J = 8.3 Hz, 2H), 7.47 (m, 4H), 7.42 (d, J = 8.3 Hz, 2H), 7.30 (m, 4H), 7.18 (m, 2H), 3.64 (s, 3H), 2.98 (m, 4H), 2.42 - 2.40 (m, 4H), 1.96 (m, 4H), 1.62 (s, 6H), 1.42 (m, 4H).

### Step 2: Preparation of 2-(4-(4-(4-(diphenylmethoxy) piperidin-1-yl) butyryl) phenyl) -2-methylpropanoic acid (carebastine)

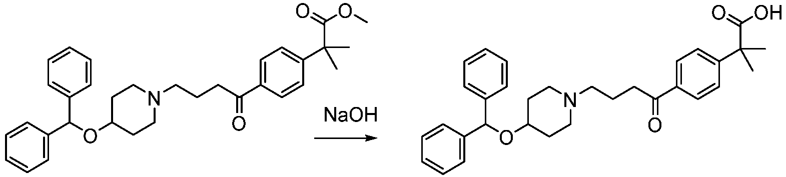

In a 25ml three-necked flask, (5-methyl-2-oxo-1,3-dioxo-4-yl) methyl-2-(4-(4-(4-(diphenylmethoxy) piperidin-1-yl) -butyryl) phenyl) -2-methylpropionic acid methyl ester (320 mg, 0.62 mmol), 1.5 ml of methanol, and 2ml of 10% NaOH were added, and the mixture was heated to 60°C and reacted for 2 h. The completion of the reaction of the raw materials was detected by TLC. After the reaction was completed, the mixture was cooled to the room temperature and concentrated to dryness, and EA was added. The pH was adjusted to 2 to 3 with hydrochloric acid. The mixture was separated into layers, washed with water once, and the organic phase was dried and concentrated to obtain 300 mg of title compound with a yield of 95% and a purity of 95.0%.

ESI-MS: m/z = 500.3(M+H)⁺.

¹H NMR (400 MHz, CDCl₃) δ: 7.75 - 7.63 (m, 2H), 7.57 - 7.24 (m, 12H), 5.48 (s, 1H), 3.73 (m, 1H), 3.05 - 3.02 (m, 2H), 2.77 - 2.66 (m, 6H), 2.20 - 2.07 (m, 2H), 2.00 - 1.81 (m, 4H), 1.58 (s, 6H).

### Step 3: Preparation of potassium 2-(4-(4-(4-(diphenylmethoxy) piperidin-1-yl) butyryl) phenyl) -2-methylpropanoate (carebastine potassium salt)

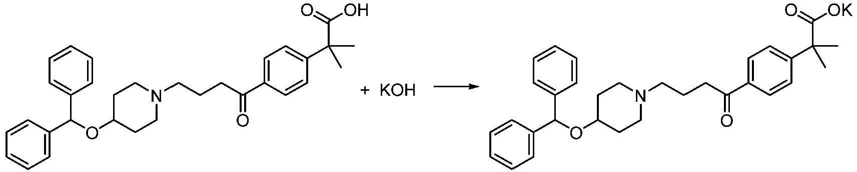

In a 25 ml three-necked flask, 2-(4-(4-(4-(diphenylmethoxy) piperidin-1-yl) butyryl) phenyl) -2-methylpropanoic acid (499 mg, 1 mmol) and 3.5 ml of acetonitrile were added and heated to 60°C. Then, potassium hydroxide (56 mg, 1 mmol) was added, stirred and cooled to precipitate a white solid, which was filtered and dried to obtain 500 mg of a carebastine potassium salt with a yield of 90% and a purity of 98.67%.

ESI-MS: m/z = 500.3(M+H)⁺.

¹H NMR (400 MHz, CDCl₃) δ: 7.75 - 7.63 (m, 2H), 7.57 - 7.24 (m, 12H), 5.48 (s, 1H), 3.73 (m, 1H), 3.05 - 3.02 (m, 2H), 2.77 - 2.66 (m, 6H), 2.20 - 2.07 (m, 2H), 2.00 - 1.81 (m, 4H), 1.58 (s, 6H).

### Example 2: Preparation of sodium 2-(4-(4-(4-(diphenylmethoxy) piperidin-1-yl) butyryl) phenyl) -2-methylpropanoate (carebastine sodium salt)

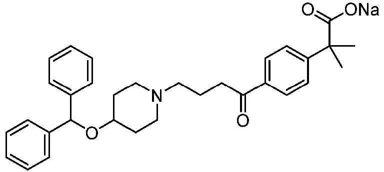

The method for preparing 2-(4-(4-(4-(diphenylmethoxy) piperidin-1-yl) butanoyl) phenyl) -2-methylpropionic acid in this example is the same as that in Example 1.

In a 25 ml three-necked flask, 2-(4-(4-(4-(diphenylmethoxy) piperidin-1-yl) butyryl) phenyl) -2-methylpropanoic acid (499 mg, 1 mmol) and 3.5 ml of acetonitrile were added and heated to 60°C. Then, sodium hydroxide (40 mg, 1 mmol) was added and stirred for 1 h, and the mixture was concentrated to dryness. After that, methyl tert-butyl ether was added and stirred, and the mixture was filtered and dried to obtain 458 mg of a carebastine sodium salt with a yield of 85% and a purity of 96.98%.

ESI-MS: m/z = 500.3(M+H)⁺.

¹H NMR (400 MHz, CDCl₃) δ: 7.75 - 7.63 (m, 2H), 7.57 - 7.24 (m, 12H), 5.48 (s, 1H), 3.73 (m, 1H), 3.05 - 3.02 (m, 2H), 2.77 - 2.66 (m, 6H), 2.20 - 2.07 (m, 2H), 2.00 - 1.81 (m, 4H), 1.58 (s, 6H).

### Example 3: Preparation of 2-(4-(4-(4-(diphenylmethoxy) piperidin-1-yl) butyryl) phenyl) -2-methylpropanoic acid p-toluenesulfonate (carebastine p-toluenesulfonate)

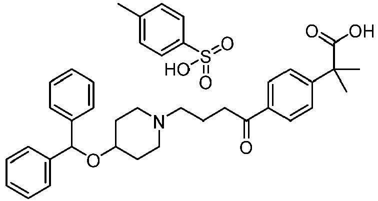

The method for preparing 2-(4-(4-(4-(diphenylmethoxy) piperidin-1-yl) butanoyl) phenyl) -2-methylpropionic acid in this example is the same as that in Example 1.

In a 25 ml three-necked flask, 2-(4-(4-(4-(diphenylmethoxy) piperidin-1-yl) butyryl)phenyl) -2-methylpropanoic acid (500 mg, 0.1 mmol) and 1 ml of isopropanol were added and heated to 60°C. Then, p-toluenesulfonic acid (172 mg, 1 mmol) was added, stirred and cooled to precipitate a white solid, which was filtered and dried to obtain 585 mg of a carebastine p-toluenesulfonate with a yield of 87% and a purity of 99.99%.

ESI-MS: m/z = 500.3(M+H)⁺.

¹H NMR (400 MHz, CDCl₃) δ: 7.94 (d, 2H), 7.49 (dd, 4H), 7.36 (dt, 8H), 7.29 - 7.21 (m, 2H), 7.11 (d, 2H), 5.48 (s, 1H), 3.73 (m, 1H), 3.05 - 3.02 (m, 2H), 2.77 - 2.66 (m, 6H), 2.41 (s, 3H), 2.20 - 2.07 (m, 2H), 2.00 - 1.81 (m, 4H), 1.58 (s, 6H).

### Example 4: Preparation of 2-(4-(4-(4-(diphenylmethoxy) piperidin-1-yl) butyryl) phenyl) -2-methylpropanoic acid methanesulfonate (carebastine methanesulfonate)

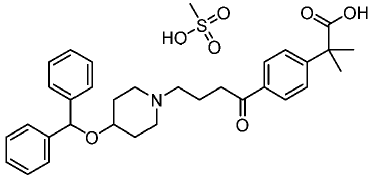

The method for preparing 2-(4-(4-(4-(diphenylmethoxy) piperidin-1-yl) butanoyl) phenyl) -2-methylpropionic acid in this example is the same as that in Example 1.

In a 25 ml three-necked flask, 2-(4-(4-(4-(diphenylmethoxy) piperidin-1-yl) butyryl) phenyl) -2-methylpropanoic acid (499 mg, 1 mmol) and 3.5 ml of EA were added and heated to 60°C. Then, methanesulfonic acid (96 mg, 1 mmol) was added and stirred for 1 h, and the mixture was concentrated to dryness. After that, methyl tert-butyl ether was added and stirred, and the mixture was filtered and dried to obtain a white solid, 482 mg of carebastine methanesulfonate, with a yield of 81% and a purity of 96.81 %.

ESI-MS: m/z = 500.3(M+H)⁺.

¹H NMR (400 MHz, CDCl₃) δ: δ: 7.75 - 7.63 (m, 2H), 7.57 - 7.24 (m, 12H), 5.48 (s, 1H), 3.73 (m, 1H), 3.29 (s, 3H), 3.09 (s, 1H), 3.05 - 3.02 (m, 2H), 2.77 - 2.66 (m, 6H), 2.20 - 2.07 (m, 2H), 2.00 - 1.81 (m, 4H), 1.58 (s, 6H).

### Example 5: Preparation of carebastine p-toluenesulfonate

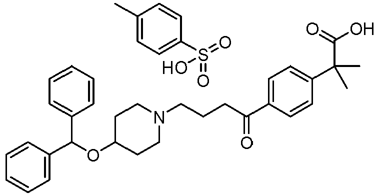

In a 25 ml three-necked flask, 2-(4-(4-(4-(diphenylmethoxy) piperidin-1-yl) butyryl) phenyl) -2-methylpropanoic acid (500 mg, 0.1 mmol) and 1 ml of isopropanol were added and heated to 80°C. Then, p-toluenesulfonic acid (172 mg, 1 mmol) was added and stirred until the reaction was completed. The mixture was cooled, crystallized, filtered, and dried to obtain 565 mg of carebastine p-toluenesulfonate with a yield of 84% and a purity of 97.91%.

ESI-MS: m/z = 500.3(M+H)⁺.

¹H NMR (400 MHz, CDCl₃) δ: 7.94 (d, 2H), 7.49 (dd, 4H), 7.36 (dt, 8H), 7.29 - 7.21 (m, 2H), 7.11 (d, 2H), 5.48 (s, 1H), 3.73 (m, 1H), 3.05 - 3.02 (m, 2H), 2.77 - 2.66 (m, 6H), 2.41 (s, 3H), 2.20 - 2.07 (m, 2H), 2.00 - 1.81 (m, 4H), 1.58 (s, 6H).

Note: In each test example of the present invention, "ebastine (Comparative Example 1 group, purchased)" indicates that the test sample used in this group is ebastine (Comparative Example 1) purchased from the market, and "carebastine p-toluenesulfonate (Example 3 group)" indicates that the test sample used in this group is the carebastine p-toluenesulfonate prepared in Example 3. Similar records shall be interpreted similarly.

### Test Example 1: Histamine-induced shock test in guinea pigs

### 1. Test sample

Ebastine (Comparative Example 1 group, purchased), carebastine potassium salt (Example 1 group), carebastine sodium salt (Example 2 group), carebastine p-toluenesulfonate (Example 3 group), and carebastine methanesulfonate (Example 4 group).

### 2. Test method

Sixty guinea pigs weighing about 220 g were randomly divided into 6 groups, namely, blank control group, Example 1 group, Example 2 group, Example 3 group, Example 4 group, and ebastine group. The corresponding drugs were given by gavage at an equimolar dose conversion of 1.00 mg/kg of ebastine in each group except the blank control group, which was given an equal volume of 0.5% CMC-Na solution in a volume of 10 ml/kg. One hour after administration, 0.125 wt.% histamine phosphate saline solution was injected intravenously into lateral sides of hind paws of the guinea pigs at 2ml/kg. After the injection was completed within 10 s, a stopwatch was used immediately to observe the latency period for the guinea pigs to develop accelerated respiration and collapse with convulsions.

### 3. Test result

**Table 1 Results of antagonistic effect on histamine-induced shock in guinea pigs (x±s, n=10)**

| **Group** | **Dose (mg/kg)** | **Time of accelerated respiration (s)** | **Time of convulsion and collapse (s)** | **Mortality rate** |
|---|---|---|---|---|
| Blank control group | 0 | 22.23±2.31 | 30±3.77 | 5/10 |
| Example 1 group | 1.14 | 294±24.42^{*▲} | >600^{*▲} | 0/10 |
| Example 2 group | 1.11 | 275±31.41^{***▲**} | >600^{*▲} | 0/10 |
| Example 3 group | 1.44 | 351±38.16^{*▲} | >600^{*▲} | 0/10 |
| Example 4 group | 1.27 | 256±24.05^{*▲} | >600^{*▲} | 0/10 |
| Comparative Example 1 group | 1.00 | 151±19.97* | 349* | 0/10 |

Compared with the blank control group: *p < 0.01. Compared with the ebastine group (Comparative Example 1 group): ^{▲}p < 0.01.

Compared with the blank control group, after intravenous injection of histamine, both the compounds in the Examples and ebastine significantly prolonged the latency of accelerated respiration and latency of convulsion in guinea pigs sensitized to histamine (p < 0.01), and no animal death occurred. Compared with the ebastine-administered group, the compounds of groups of Examples 1, 2, 3, and 4 significantly prolonged the latency of accelerated respiration and the latency of convulsion in guinea pigs (p < 0.01).

### Test Example 2: Pharmacokinetic test in rats

### 1. Test sample

Ebastine (Comparative Example 1 group), carebastine (Comparative Example 2 group), carebastine potassium salt (Example 1 group), and carebastine p-toluenesulfonate (Example 3 group).

### 2. Test method

Twenty-four SD rats weighing about 180 g to 200 g were randomly divided into 4 groups, with 6 rats in each group, half of which were male and half were female. The rats were given the corresponding drugs by intragastric administration. The rats were fasted for 12 hours before administration, but were allowed to drink water freely. Each group was given an equimolar dose of 10 mg/kg of ebastine. The groups and doses were as follows.
Comparative Example 1 group: ebastine, 10 mg/kg;
Comparative Example 2: carebastine, 10.63 mg/kg;
Example 1 group: carebastine potassium salt, 11.11 mg/kg;
Example 3 group: carebastine p-toluenesulfonate, 14.39 mg/kg.

Preparation of test substances: The preparation of test substances was carried out on the workbench in the related art in the preparation room. DMSO was used as the solvent to prepare 1.00 mg/mL of ebastine, carebastine, Example 1 and Example 3 stock solutions. For rat administration solutions, 0.5% CMC-Na solution was used to prepare 10 mg/mL of ebastine solution, 10 mg/mL of carebastine solution, 11.11 mg/kg of Example 1 solution, and 14.39 mg/mL of Example 3 solution.

Blank blood was collected before administration, and blood was collected at predetermined time points of 0.25 h, 0.5 h, 0.75 h, 1 h, 2 h, 3 h, 4 h, 6 h, 8 h, 10 h, and 24 h after administration. About 0.5 mL of blood was collected and placed in an EDTA-K2 tube, and the plasma was separated by centrifugation and stored at - 80°C. The prototype and the metabolite, namely, carebastine, in a plasma sample were detected and analyzed by liquid chromatography-mass spectrometry (LC-MS/MS), and pharmacokinetic parameters were calculated.

### 3. Test result

After a single administration of Example 1, Example 3, ebastine, and carebastine compounds to SD rats, the content of the prototype in the ebastine group was lower than 3 ng/mL, indicating that ebastine can be rapidly metabolized after entering the body.

After a single administration of Example 1, Example 3, ebastine, and carebastine compounds to SD rats, the average pharmacokinetic parameters of carebastine metabolized in each group are shown in the following table.

**Table 2 Pharmacokinetic test results**

| **Parameter** | **Unit** | **Example 1 group** | **Example 3 group** | **Comparati ve Example 1 group** | **Comparati ve Example 2 group** |
|---|---|---|---|---|---|
| T_{1/2} | h | 4.78 | 4.70 | 1.89 | 2.9 |
| Tₘₐₓ | min | 1.5 | 0.54 | 3.0 | 1.7 |
| Cₘₐₓ | mg/L | 214 | 2383 | 117 | 141 |
| AUCₗₐₛₜ | h*ug/L | 2048 | 8170 | 593 | 895 |

As can be seen from the above table, after the rats were orally administered with the compounds of Example 1 and Example 3, the amount of carebastine in the plasma of the rats in Example 1 and Example 3, AUCₗₐₛₜ, was significantly better than that of the ebastine group and the carebastine group. However, the time to peak Tₘₐₓ of carebastine in Example 1 and Example 3 was shorter than that of the ebastine group and the carebastine group. This indicates that Example 1 and Example 3 groups of the present invention can quickly enter the body and inhibit histamine H1 receptors, and have the potential to be used in the treatment of patients with acute allergic disease such as acute urticaria in clinical practice.

In addition, during the pharmacokinetic study in rats, it was found that AUC and Cₘₐₓ of three female rats were lower than those of three male rats in the ebastine group, and there was a significant difference. This indicates that after administration of ebastine, ebastine showed gender differences in SD rats, while the compounds of the examples of the present invention did not show gender differences.

The results show that, when administered at equimolar doses, the compounds of the examples of the present invention have a shorter time to peak (Tₘₐₓ) than ebastine, especially the compound of Example 3, which has a very rapid onset of action. In addition, the compounds of the present invention do not show gender differences, have the potential to be used in the treatment of patients with an acute allergic disease such as acute urticaria in clinical practice, and can reduce individual differences in medication.

### Test Example 3: Acute toxicity study in mice

### 1. Test sample

Ebastine (Comparative Example 1), carebastine potassium salt, and carebastine p-toluenesulfonate.

### 2. Test method

Seventy healthy adult KM mice weighing 18 g to 22 g were randomly divided into 7 groups with half male and half female in each group, namely, a blank control group, a high-dose group of Example 1, a low-dose group of Example 1, a high-dose group of Example 3, a low-dose group of Example 3, a high-dose group of ebastine, and a low-dose group of ebastine.

The high-dose group of Example 1, the high-dose group of Example 3, and the high-dose group of ebastine were administered at the same mole level, which were 5.38 g/kg, 6.72 g/kg, and 5 g/kg, respectively. The low-dose group of Example 1, the low-dose group of Example 3, and the low-dose group of ebastine were administered at the same mole level, which were 2.69 g/kg, 3.36 g/kg, and 2.5 g/kg, respectively. All drugs were administered by intragastric administration, and the blank control group was administered with an equal volume of 0.5 % CMC-Na solution. All animals were fasted for 12 hours before administration, but were allowed to drink water freely.

After administration, the mice were kept and observed for 14 days, and the toxic reactions (such as death) of the mice were observed and recorded every day. After the observation period, all surviving animals were dissected to observe whether there were obvious lesions in each organ.

### 3. Test result

After a single intragastric administration of a low dose of the compounds of Examples 1 and 3, and ebastine, no obvious abnormalities were observed in all animals during the entire observation period. After the animals were dissected, no obvious abnormalities were observed in the gross anatomical observation, and MDT > 2.5 g/kg. After a single intragastric administration of a high dose of the compounds of Examples 1 and 3, and ebastine, the mortality rate in the ebastine group was higher than that in the Examples 1 and 3 groups, indicating that the compounds of Examples 1 and 3 of the present invention are safer than ebastine.

**Table 3 Statistics of mouse survival and mortality rate**

| **Test group** | **Number of surviving mice** | **Mortality rate of mice** |
|---|---|---|
| Blank control group | 10 | 0 |
| High-dose group of Example 1 | 8 | 20% |
| Low-dose group of Example 1 | 10 | 0 |
| High-dose group of Example 3 | 9 | 10% |
| Low-dose group of Example 3 | 10 | 0 |
| High-dose group of ebastine | 4 | 60% |
| Low-dose group of ebastine | 10 | 0 |

### Test Example 4: Stability Study

### 1. Test sample

Carebastine (Comparative Example 2, Comparative Example 2'), carebastine potassium salt (Example 1), and carebastine p-toluenesulfonate (Example 3, Example 5).

### 2. Test method

Three samples of each of carebastine (Comparative Example 2), refined carebastine (Comparative Example 2'), carebastine potassium salt (Example 1), carebastine p-toluenesulfonate (Example 3), and carebastine p-toluenesulfonate (Example 5) were weighed and placed in weighing flasks. The samples of each group were placed under high temperature (60°C), high humidity (RH80%), and light (5000Lux) conditions, and the samples were taken for testing after 12 days. Note: The refined carebastine of Comparative Example 2' can be prepared by column chromatography of Comparative Example 2 using common technical means in the related art.

Detection method: An appropriate amount of each of the samples was weighed accurately, and is dissolved in acetonitrile and quantitatively diluted to a solution containing about 0.08 mg per 1 ml. The detection was carried out using high performance liquid chromatography, octadecylsilane bonded silica gel as a filler, and water-acetonitrile (1:1) as a mobile phase, under a detection wavelength of 256 nm, column temperature of 35°C, and injection volume of 10 µL. Each sample solution was injected into the liquid chromatograph and the chromatogram was recorded.

### 3. Test result

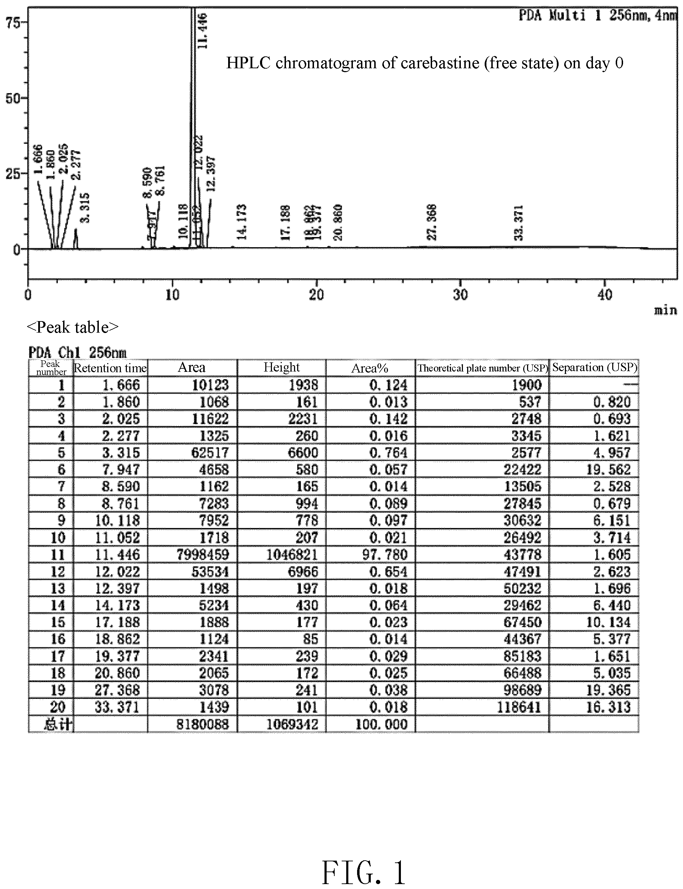

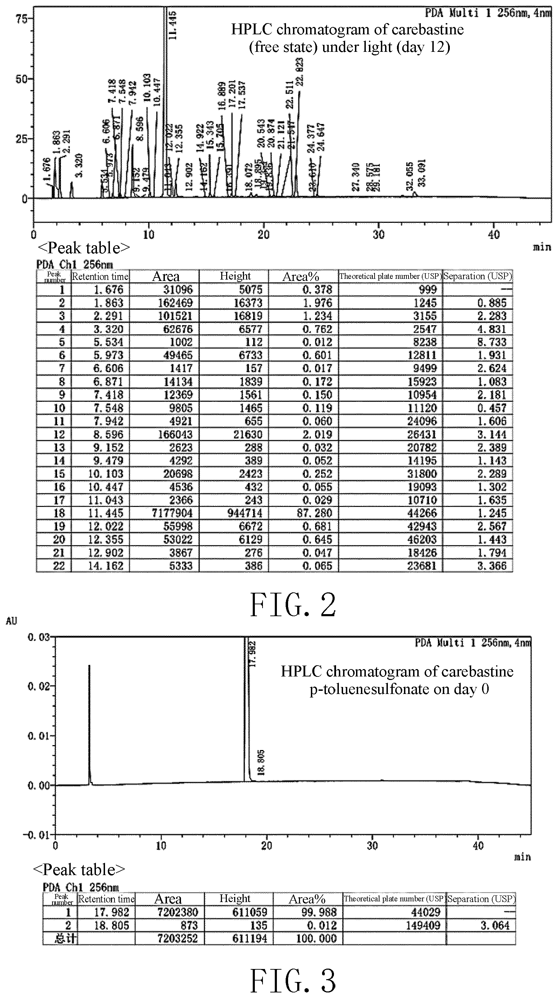

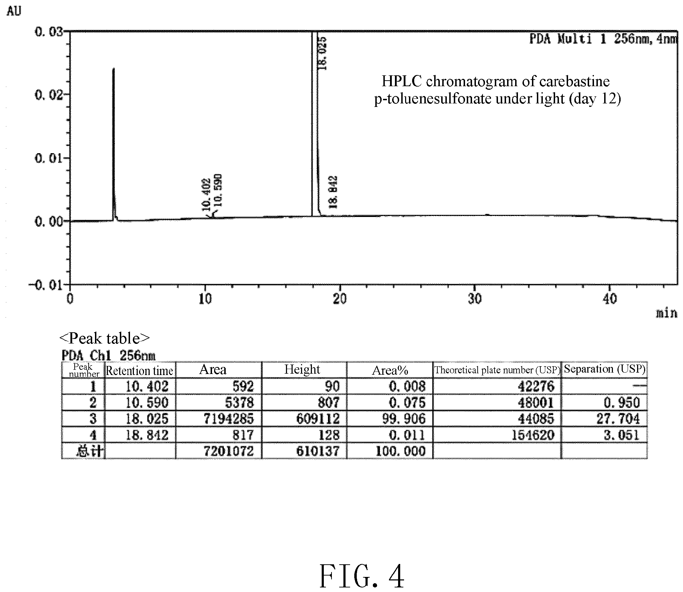

As shown in the following table, carebastine p-toluenesulfonate is stable under high temperature, high humidity and light conditions. Compared with carebastine p-toluenesulfonate, the stability of carebastine potassium salt under light is slightly reduced, but the free carebastine (Comparative Example 2) is extremely unstable under light conditions, with increased impurities and a significant decrease in purity. FIGS. 1 to 4 also show the HLPC chromatogram of free carebastine (Comparative Example 2) and carebastine p-toluenesulfonate on day 0 and day 12 under light, proving that carebastine (free state) degrades more under light, has stability problems, and produces more impurities greater than 0.1%, which is not suitable for medicinal use.

**Table 4 Test results of influencing factors**

| **Group** | **Purity** | | | |
|---|---|---|---|---|
| | **Day 0** | **Day 12 under high temperature** | **Day 12 under high humidity** | **Day 12 under light** |
| Comparative Example 2 | 97.78% | 97.22% | 97.41% | 87.28% |
| Comparative Example 2' | 99.65% | 99.45% | 99.25% | 88.11 % |
| Example 1 | 98.42% | 98.23% | 98.38% | 97.48% |
| Example 3 | 99.99% | 99.94% | 99.98% | 99.91% |
| Example 5 | 97.90% | 97.90% | 97.89% | 97.80% |

### Test Example 5: Hygroscopicity study

### 1. Test sample

Carebastine (Comparative Example 2), carebastine hydrochloride (Comparative Example 3), carebastine potassium salt (Example 1), and carebastine p-toluenesulfonate (Example 3). The preparation of carebastine hydrochloride refers to the description of patent US6340761, and the purity is 99% or more.

### 2. Test method

(1) One day before the test, a dry glass weighing bottle with a lid (outer diameter: 50 mm, height: 15 mm) was placed in a suitable constant temperature dryer at 25°C±1°C (with ammonium chloride or ammonium sulfate saturated solution placed at the bottom) or an artificial climate box (set temperature at 25°C±1°C, relative humidity at 80%±2%), and the weight is accurately weighed (m1).
(2) An appropriate amount of the sample was spread flat in the weighing bottle. The thickness of the sample is generally about 1 mm, and the weight was accurately weighed (m2).
(3) The weighing bottle was opened and placed with the lid under the above constant temperature and humidity conditions for 24 hours.
(4) The lid was put on the weighing bottle and the weight was accurately weighed (m3).
(5) Weight gain percentage = (m3 - m2)/(m2 - m1) × 100%.

### 3. Test result

The results of hygroscopicity are shown in the following table. The results show that the carebastine potassium salt and the carebastine p-toluenesulfonate of the present invention have better hygroscopicity characteristics than free carebastine and carebastine hydrochloride.

**Table 5 Hygroscopicity results**

| **Compound** | **Weight gain percentage (%)** | **Characteristic description** |
|---|---|---|
| Comparative Example 2 | 6 | Hygroscopic |
| Comparative Example 3 | 15.9 | Extremely hygroscopic |
| Example 1 | -0.2 | No or almost no hygroscopicity |
| Example 3 | 0.3 | Slightly hygroscopic |

According to the definition of the pharmacopoeia, the above results, namely "hygroscopic", means the weight gain due to moisture is less than 15% but not less than 2%, "extremely hygroscopic" means the weight gain due to moisture is not less than 15%, "no or almost no hygroscopicity" means the weight gain due to moisture is less than 0.2%, and "slightly hygroscopic" means the weight gain due to moisture is less than 2% but not less than 0.2%.

### Test Example 6: Solubility Test

### 1. Test sample

Carebastine (Comparative Example 2) and carebastine p-toluenesulfonate (Example 3).

### 2. Test method

Hydrochloric acid solution with a pH of 1.2: 7.65 ml of hydrochloric acid was diluted to 1000 ml with water, and shaken evenly to obtain the solution.

Phosphate buffer with a pH of 4.5: 3.40278 g of KH₂PO₄ was weighed, dissolved in water and diluted to 500 ml, and shaken evenly to obtain the solution.

Phosphate buffer with a pH of 6.8: 3.40353 g of KH₂PO₄ and 0.4428 g of sodium hydroxide were weighed, dissolved in water and diluted to 500 ml, and shaken evenly to obtain the solution.

Appropriate amounts of the compounds of Example 3 and Comparative Example 2 were respectively placed in conical flasks containing 50 ml of water, 50 ml of a hydrochloric acid solution with a pH of 1.2, 50 ml of a phosphate buffer with a pH of 4.5, and 50 ml of a phosphate buffer with a pH of 6.8, respectively, so that the solutions were supersaturated and insoluble matter was clearly visible.

The conical flasks were placed in a thermostatic water bath at 37°C and an oscillation rate of 120 r/min. Samples were taken at 24 h, 48 h, and 72 h, and detected by liquid chromatography.

### 3. Test results

**Table 6 Test results in different media**

| **Medium** | **Comparative Example 2 (mg/ml)** | | | **Comparative Example 3 (mg/ml)** | | |
|---|---|---|---|---|---|---|
| | **24h** | **48 h** | **72h** | **24h** | **48 h** | **72h** |
| Hydrochloric acid solution with a pH of 1.2 | 0.46 | 0.54 | 0.39 | 0.21 | 0.19 | 0.15 |
| Phosphate buffer with a pH of 4.5 | 0.20 | 0.19 | 0.20 | 0.04 | 0.05 | 0.05 |
| Phosphate buffer with a pH of 6.8 | 0.03 | 0.03 | 0.03 | 0.01 | 0.01 | 0.01 |
| Water | 0.72 | 0.52 | 0.52 | 0.08 | 0.08 | 0.08 |

The results are shown in the table above. In all tested media, at different time points, the solubility of Example 3 is lower than that of Comparative Example 2. However, the compound of Example 3 shows better pharmacokinetic properties in the pharmacokinetic test in rats.

The above embodiment is only one of the preferred embodiments of the present invention and should not be used to limit the protection scope of the present invention. Any technical problems solved by any changes or modifications that are made on the basis of the main design concept and spirit of the present invention without any substantive significance are still consistent with the present invention, and should be included in the protection scope of the present invention.

## Claims

1. A salt of carebastine, wherein the salt is an acid addition salt formed by carebastine and an acid, or a base addition salt formed by carebastine and a base.

2. The salt of carebastine according to claim 1, wherein the acid addition salt includes an inorganic acid addition salt or an organic acid addition salt,
preferably the organic acid addition salt is selected from the group consisting of methanesulfonate, benzenesulfonate, p-toluenesulfonate, naphthalene disulfonate, naphthalene-1-sulfonate, and naphthalene-2-sulfonate.

3. The salt of carebastine according to claim 2, wherein the acid addition salt is selected from the group consisting of methanesulfonate, benzenesulfonate, and p-toluenesulfonate.

4. The salt of carebastine according to claim 1, wherein the base addition salt includes an alkali metal salt, a substituted or unsubstituted ammonium salt, an amine salt, an alkaline amino acid salt, or a substituted or unsubstituted pyridinium salt,
preferably the base addition salt is selected from the group consisting of a lithium salt, a sodium salt, a potassium salt, a calcium salt, a magnesium salt, an aluminum salt, an iron salt, and a zinc salt.

5. The salt of carebastine according to claim 4, wherein the base addition salt is selected from the group consisting of a potassium salt and a sodium salt.

6. The salt of carebastine according to any one of claims 1 to 5, wherein the salt of carebastine is a single salt or a double salt,
wherein in the single salt, the molar ratio of carebastine to counter-ion is 1:1 to 2:1, and in the double salt, the molar ratio of carebastine to counter-ion is 1:4 to 4:1.

7. The salt of carebastine according to any one of claims 1 to 6, wherein the salt of carebastine is selected from

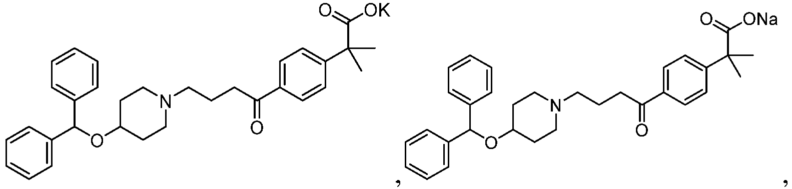

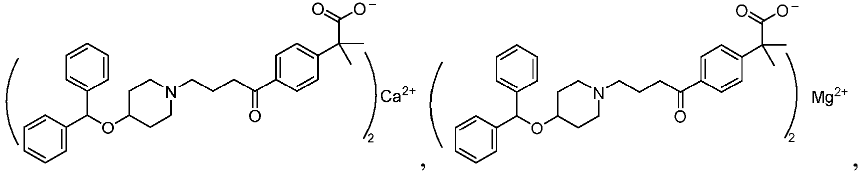

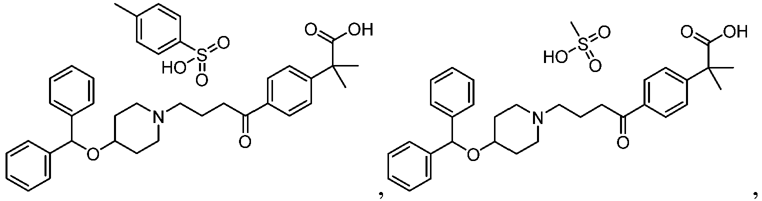

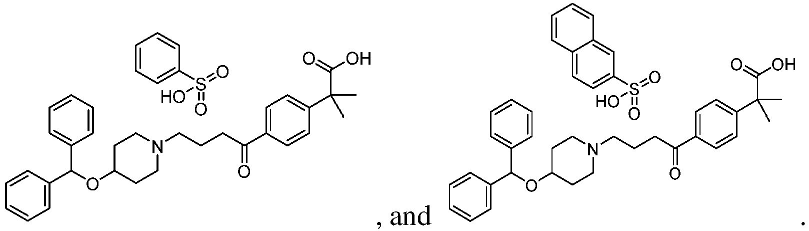

8. A method for preparing the salt of carebastine according to any one of claims 1 to 7, the method comprising: heating 2-(4-(4-(4-(diphenylmethoxy)piperidin-1-yl)butyryl)phenyl)-2-methylpropanoic acid with an acid or a base in an organic solvent to allow them to react to form a salt.

9. A pharmaceutical composition comprising: the salt of carebastine according to any one of claims 1 to 7, and a pharmaceutically acceptable carrier.

10. Use of the salt of carebastine according to any one of claims 1 to 7 in the preparation of a medicament which is a histamine H1 receptor antagonist, preferably use thereof in the preparation of a medicament for treating and/or preventing an allergic disease, more preferably, the allergic disease is an acute allergic disease, still more preferably, the allergic disease is selected from urticaria, allergic rhinitis, eczema, dermatitis, and pruritus, and even more preferably, the acute allergic disease is acute urticaria or acute allergic rhinitis.
